(19) Europäisches Patentamt / European Patent Office / Office européen des brevets

(11) **EP 3 479 044 B1**

(12) **FASCICULE DE BREVET EUROPEEN**

(45) Date de publication et mention de la délivrance du brevet:
**05.08.2020   Bulletin 2020/32**

(21) Numéro de dépôt: **17746159.7**

(22) Date de dépôt: **27.06.2017**

(51) Int Cl.:
*F28D 9/00* (2006.01)     *F25J 1/00* (2006.01)
*G06K 7/10* (2006.01)     *G16H 10/40* (2018.01)

(86) Numéro de dépôt international:
**PCT/FR2017/051709**

(87) Numéro de publication internationale:
**WO 2018/002509 (04.01.2018 Gazette 2018/01)**

(54) **ECHANGEUR DE CHALEUR COMPRENANT UN DISPOSITIF DE DISTRIBUTION D'UN MELANGE LIQUIDE/GAZ**

WÄRMETAUSCHER MIT EINER VORRICHTUNG ZUR VERTEILUNG EINES FLÜSSIGKEITS-GAS-GEMISCHES

HEAT EXCHANGER COMPRISING A DEVICE FOR DISTRIBUTING A LIQUID/GAS MIXTURE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **01.07.2016   FR 1656320**

(43) Date de publication de la demande:
**08.05.2019   Bulletin 2019/19**

(73) Titulaire: **L'Air Liquide Société Anonyme pour l'Etude et l'Exploitation des Procédés Georges Claude 75007 Paris (FR)**

(72) Inventeurs:
• **GRANADOS, Ludovic**
  **92800 Puteaux (FR)**
• **GRIGOLETTO, Philippe**
  **77270 Villeparises (FR)**
• **HAIK-BERAUD, Natacha**
  **94500 Champigny-Sur-Marne (FR)**
• **LAZZARINI, Sophie**
  **94160 Saint Mande (FR)**
• **PEYRON, Jean-Marc**
  **94000 Creteil (FR)**
• **ROSA, Eduard**
  **75015 Paris (FR)**

(74) Mandataire: **Debecker, Isabelle Virginie Air Liquide Direction de la Propriete Intellectuelle 75, Quai d'Orsay 75321 Paris Cedex 07 (FR)**

(56) Documents cités:
**EP-A2- 0 797 065     FR-A1- 2 563 620**
**GB-A- 2 127 140     US-A- 5 122 174**

EP 3 479 044 B1

**Description**

La présente invention concerne un échangeur de chaleur comprenant des séries de passages pour chacun des fluides à mettre en relation d'échange thermique, l'échangeur comprenant au moins un dispositif mélangeur configuré pour distribuer au moins un mélange à deux phases liquide/gaz dans une des séries de passages.

En particulier, la présente invention peut s'appliquer à un échangeur de chaleur qui vaporise au moins un débit de mélange liquide-gaz, en particulier un débit de mélange à plusieurs constituants, par exemple un mélange d'hydrocarbures, par échange de chaleur avec au moins un autre fluide, par exemple du gaz naturel.

La technologie couramment utilisée pour un échangeur est celle des échangeurs en aluminium à plaques et à ailettes brasés, qui permettent d'obtenir des dispositifs très compacts offrant une grande surface d'échange.

Ces échangeurs comprennent des plaques entre lesquelles sont insérées des ondes d'échange thermique, formées d'une succession d'ailettes ou jambes d'onde, constituant ainsi un empilage de passages de vaporisation et de passages de condensation, les uns destinés à vaporiser du liquide frigorigène et les autres à condenser un gaz calorigène. Les échanges de chaleur entre les fluides peuvent avoir lieu avec ou sans changement de phase.

Afin d'assurer le bon fonctionnement d'un échangeur mettant en œuvre un mélange liquide-gaz, la proportion de phase liquide et de phase gazeuse doit être la même dans tous les passages et doit être uniforme au sein d'un même passage.

Le dimensionnement de l'échangeur est calculé en supposant une répartition uniforme des phases, et donc une seule température de fin de vaporisation de la phase liquide, égale à la température de rosée du mélange.

Pour un mélange à plusieurs constituants, la température de fin de vaporisation va dépendre de la proportion de phase liquide et de phase gazeuse dans les passages.

Dans le cas d'une répartition inégale des deux phases, le profil de température du fluide frigorigène va donc varier selon les passages, voire varier au sein d'un même passage. Du fait de cette répartition non uniforme, il peut alors arriver que le ou les fluides calorigènes en relation d'échange avec le mélange à deux phases aient une température en sortie de l'échangeur supérieure à celle prévue, ce qui dégrade en conséquence les performances de l'échangeur de chaleur.

Une solution pour répartir le plus uniformément possible les phases liquide et gazeuse du mélange consiste à les introduire séparément dans l'échangeur, puis à les mélanger entre elles seulement à l'intérieur de l'échangeur.

Le document FR-A-2563620 décrit un tel échangeur dans lequel un dispositif mélangeur, tel une barre rainurée, est inséré dans la série de passages destinée à canaliser le mélange à deux phases. Le dispositif mélangeur comporte des entrées séparées pour une phase liquide et une phase gazeuse débouchant dans un volume mélangeur commun muni d'une sortie pour distribuer le mélange liquide-gaz vers la zone d'échange thermique.

Cependant, la phase liquide alimentant le dispositif mélangeur se trouve alors inévitablement en situation d'échange thermique avec le ou les fluides calorigènes circulant dans les passages adjacents de l'autre série de passages. Ceci peut engendrer un début de vaporisation de la phase liquide au sein même des entrées correspondantes, entraînant de ce fait une répartition inégale des deux phases du mélange dans certains passages de la série ainsi que dans certaines zones au sein d'un même passage.

Afin de minimiser les échanges de chaleur pouvant se produire au niveau du dispositif mélangeur, une solution serait d'installer le dispositif mélangeur dans une zone de l'échangeur dans laquelle aucun autre fluide ne circule. Il faudrait alors placer le dispositif mélangeur à une extrémité de l'échangeur, exempte de tout moyen d'évacuation ou d'amenée de fluide, ce qui imposerait de restructurer l'échangeur dans sa globalité et conduirait nécessairement à en augmenter l'encombrement. En outre, une telle solution ne permet pas l'introduction du mélange à deux phases au milieu de l'échangeur, ce qui peut être souhaitable dans des cas où les spécificités du procédé l'imposent.

La présente invention a pour but de résoudre en tout ou partie les problèmes mentionnés ci-avant, notamment en proposant un échangeur de chaleur dans lequel la répartition des phases liquide et gazeuse d'un mélange est la plus uniforme possible, et ce sans complexifier de façon excessive la structure de l'échangeur, ni en augmenter l'encombrement.

La solution selon l'invention est alors un échangeur de chaleur comprenant :

- plusieurs plaques agencées parallèlement entre elles de façon à définir une première série de passages pour canaliser au moins un fluide frigorigène et une deuxième série de passages pour canaliser au moins un fluide calorigène à mettre en relation d'échange thermique avec au moins ledit fluide frigorigène, chaque passage étant défini entre deux plaques successives et s'étendant parallèlement à un axe longitudinal,
- au moins un dispositif mélangeur agencé dans au moins un passage de la première série, ledit dispositif mélangeur étant configuré pour recevoir une phase liquide et une phase gazeuse du fluide frigorigène et distribuer un mélange desdites phases dans ledit au moins un passage,

caractérisé en ce qu'au moins un passage de la deuxième série adjacent audit au moins un passage de la première série comprend une structure d'échange thermique divisée, selon l'axe longitudinal z, en au moins une première portion et une deuxième portion juxtaposées le long de l'axe longitudinal,
la deuxième portion s'étendant en regard d'au moins une partie du dispositif mélangeur et étant configurée de manière à présenter un coefficient d'échange thermique inférieur au coefficient d'échange thermique de la première portion.

Selon le cas, l'échangeur de l'invention peut comprendre l'une ou plusieurs des caractéristiques techniques suivantes :

- lesdites première et deuxième portions comprennent respectivement au moins une première onde et au moins une deuxième onde, lesdites première et deuxième ondes étant juxtaposées le long de l'axe longitudinal z et ayant chacune plusieurs jambes d'onde reliées alternativement par des sommets d'onde et par des bases d'onde se succédant selon une direction d'ondulation, la deuxième onde s'étendant en regard d'au moins une partie du dispositif mélangeur et étant configurée de manière à présenter un coefficient d'échange thermique inférieur au coefficient d'échange thermique de la première onde.

- la deuxième onde a une densité inférieure à la densité de la première onde, la densité de chaque onde étant définie comme le nombre de jambes d'onde par unité de longueur mesuré le long de la direction d'ondulation.

- la densité de la deuxième onde représente entre 20 % et 80 %, de préférence entre 25 % et 50 %, de la densité de la première onde.

- la densité de la deuxième onde est comprise entre 6 et 15 jambes par pouce ou *fins per inch* (1 pouce=2,54 centimètres).

- lesdites première et deuxième portions sont formées à partir de feuillards, la deuxième portion étant formée à partir d'au moins un feuillard ayant une épaisseur supérieure à l'épaisseur d'au moins un feuillard formant ladite première onde de la portion.

- la deuxième onde est une onde droite, la première onde étant choisie parmi une onde droite, une onde droite perforée, une onde à décalage partiel, une onde à vagues ou une onde du type *herringbone*.

- la deuxième portion s'étend au moins en regard de la totalité du dispositif mélangeur.

- la deuxième portion présente, parallèlement à l'axe longitudinal, une longueur comprise entre 30 et 500 mm.

- la structure d'échange thermique est en outre divisée, selon l'axe longitudinal, en une troisième portion, la deuxième portion étant configurée de manière à présenter un coefficient d'échange thermique inférieur ou égal au coefficient d'échange thermique de la troisième portion, la deuxième portion étant agencée entre la première portion et la troisième portion.

- l'échangeur comprend des premiers moyens de distribution ou d'évacuation du fluide calorigène dans ledit ou dudit au moins un passage de la deuxième série de passages et des seconds moyens de distribution d'une phase liquide ou gazeuse du fluide frigorigène dans ledit au moins un passage de la première série vers le dispositif mélangeur.

- le dispositif mélangeur comprend des entrées distinctes pour une phase liquide et une phase gazeuse du fluide frigorigène, lesdites entrées distinctes étant reliées fluidiquement, via un volume mélangeur commun, à au moins une sortie pour un mélange desdites phases liquide et gazeuse.

- la sortie pour un mélange à deux phases liquide/gaz du dispositif mélangeur est située, selon l'axe longitudinal, à une première position dans ledit au moins un passage de la première série, la deuxième portion s'étendant au moins à partir de la première position jusqu'à une deuxième position située entre la première position et les premiers moyens de distribution ou d'évacuation du fluide calorigène.

- la deuxième portion s'étend jusqu'aux premiers moyens de distribution ou d'évacuation du fluide calorigène.

- la deuxième portion est formée par tout ou partie desdits premiers moyens de distribution ou d'évacuation du fluide calorigène.

La présente invention peut s'appliquer à un échangeur de chaleur qui vaporise au moins un débit de mélange liquide-gaz, en particulier un débit de mélange à plusieurs constituants, par exemple un mélange d'hydrocarbures par exemple du gaz naturel, par échange de chaleur avec au moins un autre fluide, par exemple du gaz naturel.

L'expression "gaz naturel" se rapporte à toute composition contenant des hydrocarbures dont au moins du méthane. Cela comprend une composition « brute » (préalablement à tout traitement ou lavage), ainsi que toute composition ayant été partiellement, substantiellement ou entièrement traitée pour la réduction et/ou élimination d'un ou plusieurs composés, y compris, mais sans s'y limiter, le soufre, le dioxyde de carbone, l'eau, le mercure et certains hydrocarbures lourds et aromatiques.

La présente invention va maintenant être mieux comprise grâce à la description suivante, donnée uniquement à titre d'exemple non limitatif et faite en référence aux schémas ci-annexés, parmi lesquels :

- la Figure 1 est une vue schématique en coupe, dans un plan parallèle aux axes longitudinal et transversal, d'une partie d'un passage de l'échangeur de chaleur alimenté en mélange à deux phases liquide-gaz conformément à l'invention ;

- les Figures 2A et 2B sont des vues schématiques en coupe, suivant deux plans perpendiculaires à celui de la figure 1, illustrant un exemple de structure et de fonctionnement du dispositif mélangeur d'un échangeur selon la Figure 1 ;

- la Figure 3 est une vue schématique en coupe, dans un plan parallèle à l'axe longitudinal et perpendiculaire à l'axe latéral, de séries de passages de l'échangeur de chaleur de la figure 1 selon un mode de réalisation de l'invention ;

- la Figure 4 est une autre vue schématique en coupe, dans un plan parallèle à celui de la figure 3 mais dans une direction opposée à celle de la Figure 3, de séries de passages de l'échangeur de chaleur de la figure 1 selon un autre mode de réalisation de l'invention ;

- les Figures 5A et 5B sont des vues schématiques en coupe de portions de structure d'échange thermique selon un mode de réalisation de l'invention.

L'échangeur de chaleur 1 selon l'invention comprend un empilement de plaques 2 qui s'étendent suivant deux dimensions, longueur et largeur, respectivement suivant l'axe longitudinal z et l'axe latéral y. Les plaques 2 sont disposées parallèlement l'une au-dessus de l'autre avec espacement et forment ainsi une pluralité de passages pour des fluides en relation d'échange de chaleur indirect via les plaques 2. L'axe latéral y est orthogonal à l'axe longitudinal z et parallèle aux plaques 2.

De préférence, chaque passage a une forme parallélépipédique et plate. L'écart entre deux plaques successives est petit devant la longueur et la largeur de chaque plaque successive.

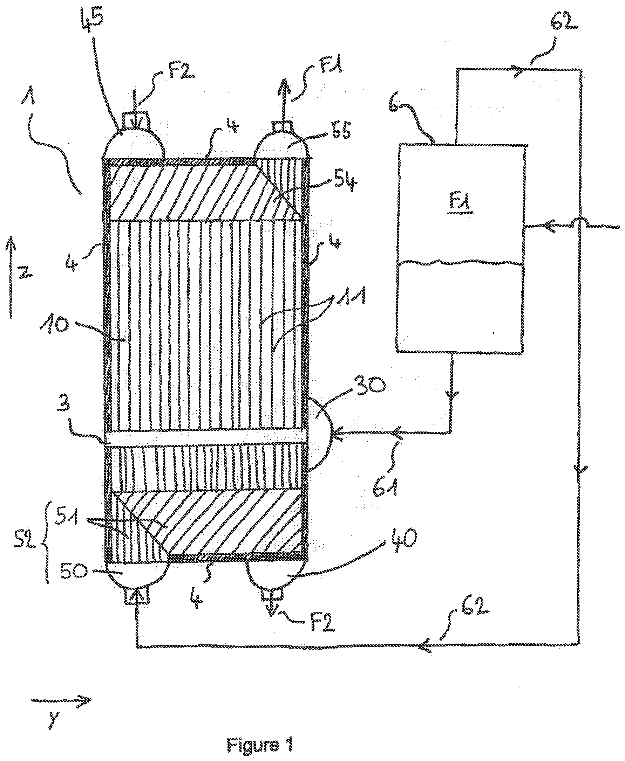

L'échangeur 1 peut comprendre un nombre de plaques supérieur à 20, voire supérieur à 100, définissant entre elles une première série de passages 10 pour canaliser au moins un fluide frigorigène F1, et une deuxième série de passages 20 (non représentée sur la Figure 1) pour canaliser au moins un fluide calorigène F2, l'écoulement desdits fluides ayant lieu globalement suivant l'axe longitudinal z. Les passages 10 de la première série peuvent être agencés, en tout ou partie, en alternance ou de façon adjacente avec tout ou partie des passages 20 de la deuxième série.

De façon connue en soi, l'échangeur 1 comprend des moyens de distribution et d'évacuation 42, 43, 52, 53 configurés pour distribuer les différents fluides sélectivement dans les passages 10, 20, ainsi que pour évacuer lesdits fluides desdits passages 10, 20.

L'étanchéité des passages 10, 20 le long des bords des plaques 2 est généralement assurée par des bandes d'étanchéité latérales et longitudinales 4 fixées sur les plaques 2. Les bandes d'étanchéité latérales 4 n'obturent pas complétement les passages 10, 20 mais laissent avantageusement des ouvertures d'entrée et de sortie de fluide situées dans les coins diagonalement opposés des passages.

Les ouvertures des passages 10 de la première série sont disposées en coïncidence l'une au-dessus de l'autre, tandis que les ouvertures des passages 20 de la deuxième série sont disposées dans les coins opposés. Les ouvertures placées l'une au-dessus de l'autre sont réunies respectivement dans des collecteurs de forme semi-tubulaire 40, 45, 50, 55, par lesquels s'effectuent la distribution et l'évacuation des fluides.

Dans les représentations des Figures 1, 3 et 4, les collecteurs semi-tubulaires 50, 45 servent à l'introduction des fluides dans l'échangeur 1 et les collecteurs semi-tubulaires 40, 55 servent à l'évacuation de ces fluides hors de l'échangeur 1.

Dans cette variante de réalisation, le collecteur d'alimentation d'un des fluides et le collecteur d'évacuation de l'autre fluide sont situés à une même extrémité de l'échangeur, les fluides F1, F2 circulant ainsi à contre-courant dans l'échangeur 1.

Selon une autre variante de réalisation, les fluides frigorigène et calorigène peuvent également circuler à co-courant, les moyens d'alimentation d'un des fluides et les moyens d'évacuation de l'autre fluide étant alors situés à des extrémités opposées de l'échangeur 1.

De préférence, l'axe longitudinal est vertical lorsque l'échangeur 1 est en fonctionnement. Le fluide frigorigène F1 s'écoule globalement verticalement et dans le sens ascendant. D'autres directions et sens d'écoulement des fluides F1, F2 sont bien entendu envisageables, sans sortir du cadre de la présente invention.

A noter que dans le cadre de l'invention, un ou plusieurs fluides frigorigènes F1 et un ou plusieurs fluides calorigènes F2 de natures différentes peuvent s'écouler au sein des passages 10, 20 des première et deuxième séries d'un même échangeur.

Les moyens de distribution et d'évacuation 42, 43, 52, 53 comprennent avantageusement des ondes de distribution 41, 44, 51, 54, agencées entre deux plaques 2 successives sous forme de tôles ondulées, qui s'étendent à partir des ouvertures d'entrée et de sortie. Les ondes de distribution 41, 44, 51, 54 assurent la répartition uniforme et la récupération des fluides sur toute la largeur des passages 10, 20.

En outre, les passages 10, 20 comprennent avantageusement des structures d'échange thermique disposées entre les plaques 2. Ces structures ont pour fonction d'augmenter la surface d'échange thermique de l'échangeur. En effet, les structures d'échange thermique sont en contact avec les fluides circulant dans les passages et transferrent des flux thermiques par conduction jusqu'aux plaques 2 adjacentes, auxquelles elles peuvent être fixées par brasage, ce qui augmente la résistance mécanique de l'échangeur.

Les structures d'échange thermique ont aussi une fonction d'entretoises entre les plaques 2, notamment lors de l'assemblage par brasage de l'échangeur et pour éviter toute déformation des plaques lors de la mise en oeuvre des fluides sous pression. Elles assurent également le guidage des écoulements de fluide dans les passages de l'échangeur.

De préférence, ces structures comprennent des ondes d'échange thermique 11 qui s'étendent avantageusement suivant la largeur et la longueur des passages 10, 20, parallèlement aux plaques 2, dans le prolongement des ondes de distribution 41, 44, 51, 54 selon la longueur des passages 10, 20. Les passages 10, 20 de l'échangeur présente ainsi une partie principale de leur longueur constituant la partie d'échange thermique proprement dite, qui est garnie d'une structure d'échange thermique, ladite partie principale étant bordée par des parties de distribution garnies des ondes de distribution 41, 44, 51, 54.

La Figure 1 illustre un passage 10 de la première série 1 configuré pour distribuer un fluide frigorigène F1 se présentant sous la forme d'un mélange liquide-gaz à deux phases. Le fluide frigorigène F1 est séparé dans un dispositif séparateur 6 en une phase liquide 61 et une phase gazeuse 62 introduites séparément dans l'échangeur 1 par l'intermédiaire d'un collecteur latéral 30 et du collecteur 50. Les deux phases 61, 62 sont ensuite mélangées l'une avec l'autre au moyen d'un dispositif mélangeur 3 agencé dans le passage 10 et représenté de façon schématique sur la Figure 1. Avantageusement, plusieurs passages 10, voire la totalité des passages 10 de la première série comporte un dispositif mélangeur 3.

Selon une variante de réalisation, le dispositif mélangeur 3 comprend des entrées distinctes 31, 32 pour les phases liquide ou gazeuse du fluide frigorigène F1. Lesdites entrées distinctes 31, 32 sont reliées fluidiquement, via un volume mélangeur commun, à au moins une sortie 33 pour un mélange à deux phases liquide/gaz. Les entrées 31, 32 et/ou les sorties 33 peuvent déboucher au niveau des faces extrêmes 35, 36 du dispositif mélangeur 3, ou en retrait vers l'intérieur du dispositif 3 par rapport audites faces 35, 36.

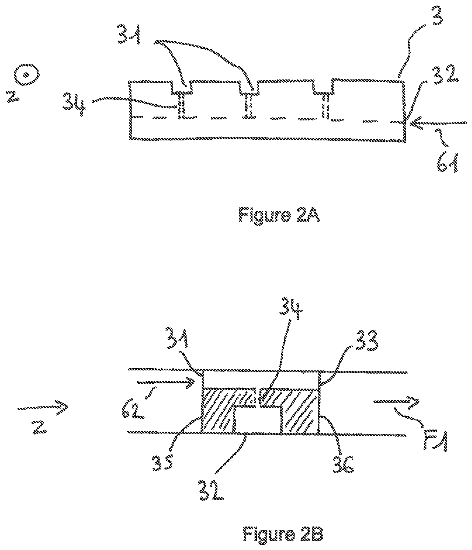

Selon le mode de réalisation illustré sur les Figures 2A et 2B, le dispositif mélangeur 3 est une barre, ou baguette, qui s'étend dans la largeur du passage 10. La barre 3 comporte des rainures 31, 32 usinées perpendiculairement les unes aux autres et reliées par des trous 34.

Dans les représentations des Figures 1, 2A et 2B, la phase gazeuse 62 est introduite dans une rangée de rainures 31 agencées parallèlement à l'axe longitudinal z et la phase liquide 61 est introduite dans au moins une rainure 32 qui s'étend parallèlement à l'axe transversal y.

Selon un autre exemple de réalisation, non illustré, le dispositif mélangeur 3 peut comprendre un ou plusieurs tubes dans lesquels est introduite une phase du fluide frigorigène F1, cette phase ressortant du ou des tubes par des orifices percés dans leur paroi. L'autre phase est introduite en amont du ou des tubes et s'écoule autour de ces tubes.

Bien entendu, les points d'introduction des phases liquide 61 et gazeuse 62 dans l'échangeur peuvent être inversés par rapport aux représentations données sur les Figures.

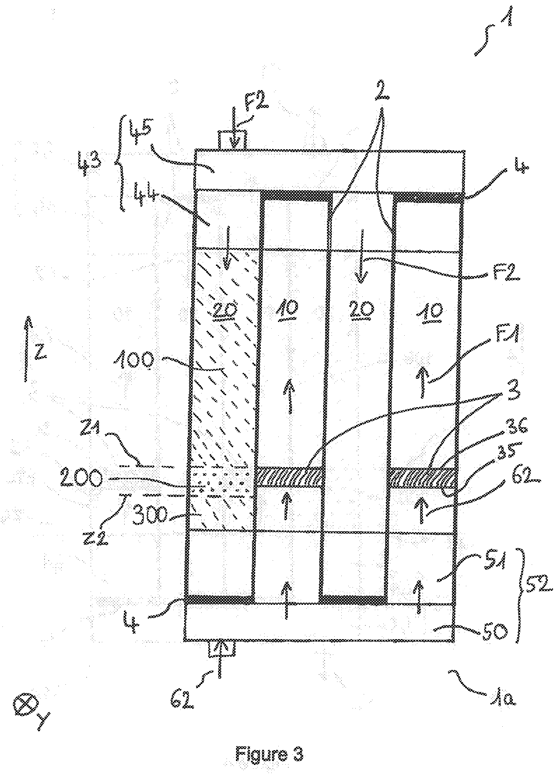

La Figure 3 est une vue schématique en coupe, dans un plan parallèle à l'axe longitudinal z et perpendiculaire à l'axe latéral y, de l'échangeur de la Figure 1. On y voit un empilement de passages 10, 20 de la première et de la deuxième séries, représentés à un nombre limité à 4 par souci de simplification.

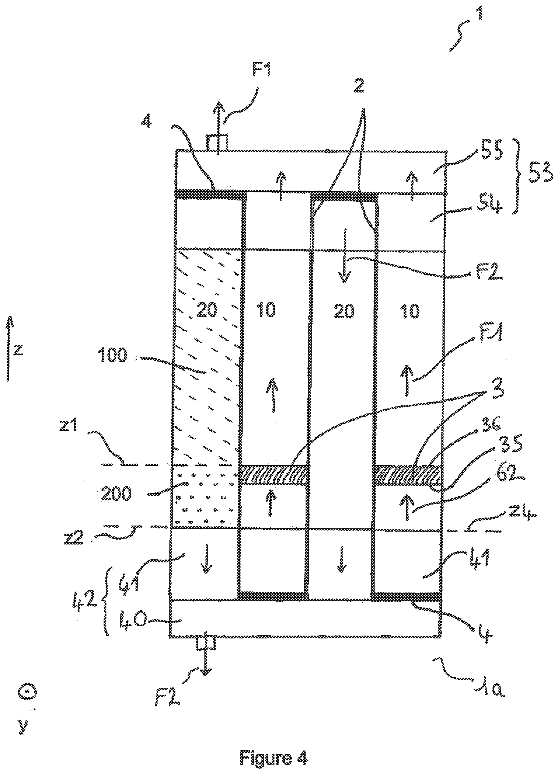

Selon l'invention, une structure d'échange thermique est logée dans au moins un passage 20 adjacent au passage 10 comprenant le dispositif mélangeur 3. Cette structure d'échange thermique est divisée, selon l'axe longitudinal z, en au moins une première portion 100 et une deuxième portion 200, la deuxième portion 200 s'étendant en regard d'au moins une partie du dispositif mélangeur 3. Les première et deuxième portions 100, 200 sont juxtaposées le long de l'axe longitudinal z de l'échangeur 1, c'est-à-dire positionnées bout à bout, comme illustré sur les Figures 3 et 4. Dit autrement, Les première et deuxième portions 100, 200 se succèdent, ou se situent immédiatement l'une à côté de l'autre, le long de l'axe z.

Les termes « en regard » signifient « en vis-à-vis » ou « au niveau» du dispositif mélangeur 3. En d'autres termes, au moins une partie de la deuxième portion 200 se situe, le long de l'axe z, à une position à laquelle se situe également une partie du dispositif mélangeur 3.

Conformément à l'invention, la deuxième portion 200 est configurée de manière à présenter un coefficient d'échange thermique inférieur au coefficient d'échange thermique de la première portion 100.

En positionnant une structure de faible efficacité thermique dans une partie du passage adjacent situé au niveau du dispositif mélangeur, on réduit grandement les échanges de chaleur pouvant avoir lieu avec le fluide calorigène au niveau des points d'entrée des phases liquide et gazeuse du fluide frigorigène dans l'échangeur. Ceci permet de limiter, voire d'éviter une vaporisation de la phase liquide du fluide frigorigène avant son mélange avec la phase gazeuse dudit fluide frigorigène. Les deux phases du mélange sont ainsi réparties de la façon la plus homogène possible au sein même des passages pour le mélange à deux phases, ainsi qu'entre les différents passages pour le mélange à deux phases.

Cette solution présente les avantages d'être simple de mise en œuvre, de ne pas modifier l'encombrement de l'échangeur et de ne pas complexifier sa structure. En outre, la structure d'échange thermique selon l'invention permet de réduire les transferts de chaleur vers le dispositif mélangeur, et ce sans affaiblir la tenue mécanique de l'échangeur, puisque la fonction d'entretoise entre les plaques 2 continue d'être assurée par la deuxième portion 200.

Par coefficient d'échange thermique, ou coefficient de transfert thermique, on entend un coefficient quantifiant le flux d'énergie traversant la structure d'échange thermique, par unité de surface, de volume ou de longueur.

On peut définir de la manière suivante le coefficient d'échange thermique (dans ce cas pour un transfert thermique surfacique) :

$$h = \frac{\Delta Q}{A.\Delta T.\Delta t}$$

avec :

- h : coefficient d'échange thermique, exprimé en watts par mètre carré-kelvin ($W \cdot m^{-2} \cdot K^{-1}$),
- $\Delta Q$ : énergie transférée en joules (J),
- A : surface d'échange en mètres carrés ($m^2$),
- $\Delta T$ : dififérence de température de part et d'autre de la surface d'échange en kelvins ou en degrés Celsius (K ou °C),
- $\Delta t$ : intervalle de temps en secondes (s).

Le coefficient de transfert thermique d'une structure dépend de paramètres intrinsèques, c'est-à-dire propres à la structure d'échange elle-même, notamment la densité de l'onde formant la structure, l'épaisseur de l'onde, ainsi que de paramètres extrinsèques, c'est-à-dire propres au procédé mis en œuvre, notamment débit des fluides et différence de température entre les fluides). La détermination du coefficient de transfert thermique se fait à l'aide du nombre de Nusselt (Nu) via la relation suivante :

$$Nu = \frac{h.L_c}{k}$$

avec :

- h : coefficient de transfert thermique,
- $L_c$, longueur caractéristique,
- k, conductivité thermique du fluide.

De nombreuses corrélations empiriques fournissent une équation pour calculer le nombre de Nusselt d'où il est possible d'extraire le coefficient de transfert thermique.

En particulier, le coefficient d'échange thermique d'une structure peut être déterminé, dans les cas de fluide monophasique liquide et gaz, par le nombre de Nusselt calculé à partir de la relation ci-dessous:

$$Nu = CjRePr^{1/3}$$

avec Nu : Nombre de Nusselt
Cj : Facteur de Colburn
Re : Nombre de Reynolds
Pr : Nombre de Prandtl

S'agissant du cas diphasique liquide-gaz, le coefficient d'échange thermique peut être déterminé à l'aide de méthodes de corrélations connues en soi.

Dans le cadre de l'invention, le coefficient d'échange thermique de la deuxième portion 200 et le coefficient d'échange thermique de la première portion 100 sont comparés à méthode de mesure ou de détermination théorique identiques ou quasi-identiques, les conditions propres au procédé d'échange (i. e. paramètres extrinsèques) étant identiques ou quasi-identiques.

De préférence, une telle structure d'échange thermique est logée dans plusieurs, voire la totalité, des passages 20 qui sont adjacents à des passages 10 comprenant un dispositif mélangeur 3. Ladite structure s'étend sur la quasi-totalité, voire la totalité, de la largeur des passages 20 selon l'axe y, de sorte que la structure est avantageusement en contact avec chaque plaque 2 formant le passage 20.

Selon un mode avantageux de réalisation de l'invention, lesdites première et deuxième portions 100, 200 de la structure d'échange thermique comprennent respectivement une première onde et une deuxième onde 100, 200. Les ondes 100, 200 comprennent chacune plusieurs jambes d'onde 123, 223, ou ailettes 123, 223, reliées alternativement par des sommets d'onde 121, 221 et par des bases d'onde 122, 222 se succédant selon des directions d'ondulation D1, D2. La deuxième onde 200 s'étend en regard d'au moins une partie du dispositif mélangeur 3 et présente une densité inférieure à la densité de la première onde. Le fait de réduire la densité de la deuxième onde 200 permet de réduire la surface d'échange de la structure en regard du dispositif mélangeur, ce qui permet de réduire les échanges thermiques par convection entre les fluides circulant dans le dispositif mélangeur et dans le passage adjacent de la deuxième série.

De préférence, la densité de la deuxième onde représente entre 10 % et 90 %, de préférence entre 20 % et 80 %, de préférence encore entre 25 et 50 %, de la densité de la première onde.

Dans le cadre de l'invention, la densité de chaque onde 100, 200 est définie comme le nombre de jambes d'onde 123, 223 par unité de longueur mesuré le long des directions d'ondulation D1, D2 respectives. Pour mesurer la densité d'une onde, on considère en général une longueur donnée de l'onde le long de sa direction d'ondulation, et on détermine le nombre de jambes d'onde se trouvant sur cette longueur, ou sur un multiple de cette longueur avec division ultérieure par ledit multiple, pour plus de précision. Généralement, la longueur considérée est un pouce, soit 2,54 cm. On parle ainsi de nombre de jambes d'onde par 2,54 cm ou de nombre d'ailettes par 2,54 cm.

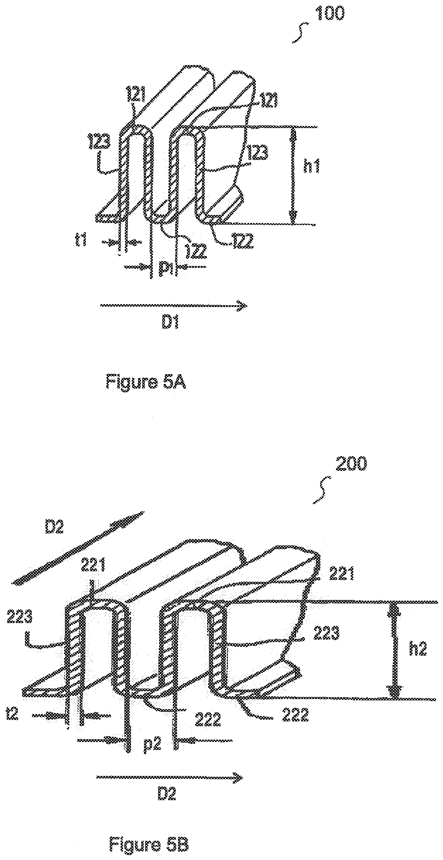

En se référant aux Figures 5A et 5B, la deuxième onde 200 présente donc une distance p2 entre deux jambes d'onde 223 successives supérieure à la distance p1 entre deux jambes d'onde 123 successives de la première onde 100.

De préférence, la densité de la deuxième onde 200 est comprise entre 6 et 15 jambes par 2,54 centimètres, c'est-à-dire 6 et 15 jambes par pouce ou *fins per inch.*

Quant à la densité de la première onde 100, elle peut être comprise entre 15 et 30 jambes par 2,54 centimètres, c'est-à-dire 15 et 30 jambes par pouce ou *fins per inch,* de préférence comprise entre 18 et 25 jambes par 2,54 centimètres, c'est-à-dire 18 et 25 jambes par pouce ou *fins per inch.*

Les Figures 5A et 5B illustrent des exemples de réalisation dans lesquels les jambes d'onde 123, 223 et les bases d'onde 122, 22 forment, en section transversale, des segments rectilignes parallèles entre eux, les jambes d'onde 123, 223 se situant dans des plans perpendiculaires aux directions D1, D2. De façon alternative, les ondes 100, 200 peuvent présenter une section transversale de forme sinusoïdale, triangulaire ou toute autre forme adéquate.

Avantageusement, les première et deuxième portions 100, 200 sont chacune formées à partir d'un ou plusieurs feuillards, c'est-à-dire de fines tôles métalliques, de préférence formés d'aluminium ou d'un alliage d'aluminium. Ces feuillards ont des épaisseurs préférentiellement comprises entre 0,2 et 0,6 mm.

Selon un mode de réalisation particulier, la deuxième portion 200 est formée à partir d'un feuillard ayant une épaisseur supérieure à l'épaisseur du feuillard formant la première portion 100. Cela permet de réduire la densité de la deuxième portion 200 tout en lui conférant une rigidité suffisante pour assurer la bonne tenue mécanique de l'échangeur.

Dans le cadre de l'invention, les première et deuxième ondes peuvent avoir des hauteurs h1, h2 typiquement comprises entre 3 et 10 mm. De préférence, les hauteurs h1, h2 sont choisies de sorte que les première et deuxième ondes 100, 200 s'étendent dans la quasi-totalité, voire la totalité, de la largeur du passage 20 selon la direction transversale y.

De préférence, la première onde 100 et/ou la deuxième onde 200 sont agencées dans les passages 10, 20 des première et deuxième séries de sorte que leurs directions d'ondulation D1, D2 soient globalement parallèles à la direction d'écoulement des fluides dans les passages 10, 20 (disposition dite « *easy way* »). Un tel agencement offre une meilleure maîtrise du procédé et limite grandement le risque de boucher les passages de l'échangeur. Ainsi, avec un agencement vertical de l'échangeur 1 conformément aux représentations des Figures 3 et 4, les ondes 100, 200 sont des ondes à génératrices verticales.

Il est aussi envisageable que la première onde 100 et/ou la deuxième onde 200 soient agencées de sorte que leurs directions d'ondulation D1, D2 soient globalement perpendiculaires à la direction d'écoulement des fluides (disposition dite « en *hard way* »). Selon les représentations des Figures 3 et 4, les ondes 100, 200 sont alors des ondes à génératrices horizontales.

On peut utiliser les différents types d'ondes mis en œuvre habituellement dans les échangeurs du type à plaques et ailettes brasés pour former les première et deuxième ondes 100, 200. Les ondes peuvent être choisie parmi les types d'onde connus tels les ondes droites, les ondes à décalage partiel (du type « *serrated* » en anglais), les ondes à vagues ou arêtes de hareng (du type « *herringbone* » en anglais), perforées ou non.

En tant que première onde, on utilisera avantageusement une onde à décalage partiel ou *herringbone*. Ce type d'onde permet de créer une turbulence dans l'écoulement du fluide calorigène F2, ce qui a pour effet d'augmenter le coefficient d'échange thermique de l'onde.

En tant que deuxième onde, on utilisera avantageusement une onde droite ou une droite perforée ce type d'onde créant moins de trubulences dans l'écoulement du fluide, et étant par conséquent moins efficace thermiquement.

La première portion 100 se situe avantageusement, le long d'une direction parallèle à la direction d'écoulement du fluide frigorigène F1 dans le passage 10, en aval de la deuxième portion 200, comme illustré sur les Figures 3 et 4. De préférence, la deuxième portion 200 s'étend en regard de la totalité du dispositif mélangeur 3. Elle peut en outre s'étendre au-delà de l'une et/ou l'autre des faces extrêmes 35, 36 du dispositif mélangeur 3. Avantageusement, la deuxième portion 200 s'étend au moins au-delà de la face extrême 35 par laquelle est amenée la phase liquide ou gazeuse du fluide frigorigène F1, comme représenté sur la Figure 4.

De cette façon, on réduit le plus possible les échanges de chaleur pouvant avoir lieu entre le fluide calorigène F2 circulant dans le passage 20 adjacent et la phase liquide du fluide frigorigène F1 avant son mélange avec la phase gazeuse.

Ainsi, dans la représentation donnée sur les Figures, la deuxième portion 200 s'étend, en suivant la direction d'écoulement du fluide calorigène F2 lors du fonctionnement de l'échangeur, en aval du dispositif mélangeur 3.

Le dispositif mélangeur 3 peut présenter, parallèlement à l'axe longitudinal z, une longueur comprise entre 30 et 80 mm.

De préférence, la deuxième portion 200 présente, parallèlement à l'axe longitudinal z, une longueur au moins égale à la longueur du dispositif mélangeur 3. La deuxième portion 200 peut présenter, parallèlement à l'axe longitudinal z, une longueur comprise entre 30 et 300 mm.

Comme expliqué précedemment, l'échangeur 1 comprend des moyens de distribution et d'évacuation 42, 43, 52, 53 configurés pour distribuer les différents fluides sélectivement dans les passages 10, 20, ainsi que pour évacuer lesdits fluides desdits passages 10, 20.

En particulier, l'échangeur 1 peut comprendre à une extrémité 1a, qui est située dans la partie basse de l'échangeur dans le cas où l'échangeur fonctionne en position verticale et que le fluide frigorigène F1 circule dans le sens ascendant (comme illustré sur les Figures 1, 3 et 4), des seconds moyens 52 de distribution d'une phase liquide ou gazeuse du fluide frigorigène F1 dans le ou les passages 10.

La même extrémité 1a comprend en outre des premiers moyens 42 qui peuvent être selon le cas, des moyens de distribution ou d'évacuation du fluide calorigène F2 dans ledit ou dudit au moins un passage 20.

Dans la configuration d'écoulement illustrée sur les Figures 1, 3 et 4, dans laquelle les fluides F1, F2 circulent à contre-courant, les premiers moyens 42 sont des moyens d'évacuation du fluide calorigène F2.

Comme schématisé sur les Figures 3 et 4, ladite au moins une sortie 33 pour le mélange des phases liquide 61 et gazeuse 62 est située, selon l'axe longitudinal z, à une première position z1 dans le passage 10 de la première série.

Avantageusement, la deuxième portion 200 s'étend au moins à partir de la première position z1 jusqu'à une deuxième position z2 située entre la première position z1 et les premiers moyens 42 de distribution ou d'évacuation du fluide calorigène F2. De cette façon, on limite au maximum la quantité de chaleur pouvant être transférée du fluide calorigène F2 vers l'une ou l'autre des deux phases tant que celles-ci ne sont pas mélangées par le dispositif 3, et on favorise les échanges de chaleur entre les fluides en amont du dispositif 3, une fois que les deux phases ont été mélangées, c'est-à-dire dans la partie principale de la longueur de l'échangeur constituant la partie d'échange thermique proprement dite.

Ainsi, la première portion 100 s'étend avantageusement, parallèlement à l'axe z, entre la deuxième portion 200 et les seconds moyens d'évacuation 53 du fluide frigorigène F1.

Selon un mode de réalisation particulier, la position z2 peut correspondre à la position de l'entrée 31 pour l'une ou l'autre des phases du fluide frigorigène F1 le long de l'axe z.

En particulier, la position z1 peut correspondre à la position de la face extrême 36 du dispositif mélangeur 3 et/ou la position z2 peut correspondre à la position de la face extrême 35 du dispositif mélangeur 3.

Avantageusement, la deuxième portion 200 s'étend, parallélement à l'axe z, jusqu'aux premiers moyens 42 de distribution ou d'évacuation du fluide calorigène F2.

En particulier, la deuxième portion 200 peut être formée par tout ou partie desdits premiers moyens 42 de distribution ou d'évacuation du fluide calorigène F2.

Dans le cas où la structure d'échange thermique comprend une deuxième portion 200 formée d'une onde 200, l'onde 200 est avantageusement formée par un prolongement de l'onde de distribution 41 dans la partie principale de la longueur de l'échangeur constituant la partie d'échange thermique proprement dite.

Selon une variante de réalisation schématisée sur la Figure 3, la structure d'échange thermique peut en outre être divisée, selon l'axe longitudinal z, en une troisième portion 300, la deuxième portion 200 étant agencée entre la première portion 100 et la troisième portion 300.

Conformément à l'invention, la deuxième portion 200 est configurée de manière à présenter un coefficient d'échange inférieur au coefficient d'échange thermique de la troisième portion 300. En particulier, la troisième portion 300 peut comprendre une troisième onde 300 de caractéristiques identiques à la deuxième onde 200.

Bien entendu, l'invention n'est pas limitée aux exemples particuliers décrits et illustrés dans la présente demande. D'autres variantes ou modes de réalisation à la portée de l'homme du métier peuvent aussi être envisagés sans sortir du cadre de l'invention comme défini dans les revendications.

Par exemple, dans la configuration d'échangeur illustrée sur les Figures, la phase liquide du fluide F1 est injectée latéralement et la phase gazeuse est injectée au niveau de l'extrémité inférieure de l'échangeur. D'autres configurations d'injection sont bien sûr envisageables, selon la proportion liquide-gaz dans le mélange à deux phases ou selon les contraintes imposées par le procédé à mettre en œuvre. Ainsi, on pourrait envisager d'injecter la phase gazeuse latéralement, en amont ou en aval du point d'injection latéral de la phase liquide.

**Revendications**

1. Echangeur de chaleur (1) comprenant :

   - plusieurs plaques (2) agencées parallèlement entre elles de façon à définir une première série de passages (10) pour canaliser au moins un fluide frigorigène (F1) et une deuxième série de passages (20) pour canaliser au moins un fluide calorigène (F2) à mettre en relation d'échange thermique avec au moins ledit fluide frigorigène (F1), chaque passage (10, 20) étant défini entre deux plaques (2) successives et s'étendant parallèlement à un axe longitudinal (z), et
   - au moins un dispositif mélangeur (3) agencé dans au moins un passage (10) de la première série, ledit dispositif mélangeur (3) étant configuré pour recevoir une phase liquide (61) et une phase gazeuse (62) du fluide frigorigène (F1) et distribuer un mélange desdites phases (61, 62) dans ledit au moins un passage (10),
   - au moins un passage (20) de la deuxième série adjacent audit au moins un passage (10) de la première série comprenant une structure d'échange thermique divisée, selon l'axe longitudinal (z), en au moins une première portion (100) et une deuxième portion (200) juxtaposées le long de l'axe longitudinal (z), la deuxième portion (200) s'étendant en regard d'au moins une partie du dispositif mélangeur (3),

**caractérisé en ce que** la deuxième portion (200) est configurée de manière à présenter un coefficient d'échange thermique inférieur au coefficient d'échange thermique de la première portion (100).

2. Echangeur selon la revendication 1, **caractérisé en ce que** lesdites première et deuxième portions (100, 200) comprennent respectivement au moins une première onde et au moins une deuxième onde, lesdites première et deuxième ondes étant juxtaposées le long de l'axe longitudinal (z) et ayant chacune plusieurs jambes d'onde (123, 223) reliées alternativement par des sommets d'onde (121, 221) et par des bases d'onde (122, 222) se succédant selon une direction d'ondulation (D1, D2), la deuxième onde s'étendant en regard d'au moins une partie du dispositif mélangeur (3) et étant configurée de manière à présenter un coefficient d'échange thermique inférieur au coefficient d'échange thermique de la première onde.

3. Echangeur selon la revendication 2, **caractérisé en ce que** la deuxième onde a une densité inférieure à la densité de la première onde, la densité de chaque onde étant définie comme le nombre de jambes d'onde par unité de longueur mesuré le long de la direction d'ondulation (D1, D2).

4. Echangeur selon la revendication 3, **caractérisé en ce que** la densité de la deuxième onde représente entre 20 % et 80 %, de préférence entre 25 % et 50 %, de la densité de la première onde.

5. Echangeur selon l'une des revendications 3 ou 4, **caractérisée en ce que** la densité de la deuxième onde est comprise entre 6 et 15 jambes par pouce (1 pouce=2,54 centimètres).

6. Echangeur selon l'une des revendications précédentes, **caractérisée en ce que** lesdites première et deuxième portions (100, 200) sont formées à partir de feuillards, la deuxième portion (200) étant formée à partir d'au moins un feuillard ayant une épaisseur supérieure à l'épaisseur d'au moins un feuillard formant ladite première onde de la portion (100).

7. Echangeur selon l'une des revendications 2 à 6, **caractérisée en ce que** la deuxième onde est une onde droite, la première onde étant choisie parmi une onde droite, une onde droite perforée, une onde à décalage partiel, une onde à vagues ou une onde du type « à arêtes de hareng ».

8. Echangeur selon l'une des revendications précédentes, **caractérisée en ce que** la deuxième portion (200) s'étend au moins en regard de la totalité du dispositif mélangeur (3).

9. Echangeur selon l'une des revendications précédentes, **caractérisée en ce que** la deuxième portion (200) présente, parallèlement à l'axe longitudinal (z), une longueur comprise entre 30 et 500 mm.

10. Echangeur selon l'une des revendications précédentes, **caractérisée en ce que** la structure d'échange thermique est en outre divisée, selon l'axe longitudinal (z), en une troisième portion (300), la deuxième portion (200) étant configurée de manière à présenter un coefficient d'échange thermique inférieur ou égal au coefficient d'échange thermique de la troisième portion (300), la deuxième portion (200) étant agencée entre la première portion (100) et la troisième portion (300).

11. Echangeur selon l'une des revendications précédentes, **caractérisé en ce qu'**il comprend, à une même extrémité de l'échangeur, des premiers moyens (42) de distribution ou d'évacuation du fluide calorigène (F2) dans ledit ou dudit au moins un passage de la deuxième série de passages (20) et des seconds moyens (52) de distribution d'une phase liquide ou gazeuse du fluide frigorigène (F1) dans ledit au moins un passage (10) de la première série vers le dispositif mélangeur (3).

12. Echangeur selon l'une des revendications précédentes, **caractérisé en ce que** le dispositif mélangeur (3) comprend des entrées distinctes (31, 32) pour une phase liquide (61) et une phase gazeuse (62) du fluide frigorigène (F1), lesdites entrées distinctes (31, 32) étant reliées fluidiquement, via un volume mélangeur commun, à au moins une sortie (33) pour un mélange desdites phases liquide et gazeuse (61, 62).

13. Echangeur selon la revendication 12, **caractérisé en ce que** la sortie (33) pour un mélange à deux phases liquide/gaz du dispositif mélangeur (3) est située, selon l'axe longitudinal (z), à une première position (z1) dans ledit au moins un passage (10) de la première série, la deuxième portion (200) s'étendant au moins à partir de la première position (z1) jusqu'à une deuxième position (z2) située entre la première position (z1) et les premiers moyens (42) de distribution ou d'évacuation du fluide calorigène (F2).

14. Echangeur selon l'une des revendications 11 à 13, **caractérisé en ce que** la deuxième portion (200) s'étend jusqu'aux premiers moyens (42) de distribution ou d'évacuation du fluide calorigène (F2).

15. Echangeur selon l'une des revendications 11 à 14, **caractérisé en ce que** la deuxième portion (200) est formée par tout ou partie desdits premiers moyens (42) de distribution ou d'évacuation du fluide calorigène (F2).

**Patentansprüche**

1. Wärmeaustauscher (1), umfassend:

   - mehrere Platten (2), die parallel zueinander angeordnet sind, um eine erste Reihe von Durchlässen (10), um mindestens ein Kältemittelfluid (F1) zu kanalisieren, und eine zweite Reihe von Durchlässen (20), um mindestens ein wärmeerzeugendes Fluid (F2) zu kanalisieren, zu definieren, das in eine Wärmeaustauschbeziehung mit mindestens dem Kältemittelfluid (F1) zu setzen ist, wobei jeder Durchlass (10, 20) zwischen zwei aufeinanderfolgenden Platten (2) definiert ist, und die sich parallel zu einer Längsachse (z) erstrecken, und
   - mindestens eine Mischvorrichtung (3), die in mindestens einem Durchlass (10) der ersten Reihe angeordnet ist, wobei die Mischvorrichtung (3) konfiguriert ist, um eine flüssige Phase (61) und eine gasförmige Phase (62) des Kältemittelfluids (F1) zu empfangen, und ein Gemisch der Phasen (61, 62) in dem mindestens einen Durchlass (10) zu verteilen,
   - mindestens einen Durchlass (20) der zweiten Reihe, angrenzend an den mindestens einen Durchlass (10) der ersten Reihe, der eine Wärmeaustauschstruktur umfasst, die entlang der Längsachse (z) in mindestens einen ersten Abschnitt (100) und einen zweiten Abschnitt (200) geteilt ist, die entlang der Längsachse (z) nebeneinandergestellt sind, wobei sich der zweite Abschnitt (200) gegenüber mindestens einem Teil der Mischvorrichtung (3) erstreckt,

   **dadurch gekennzeichnet, dass** der zweite Abschnitt (200) derart konfiguriert ist, dass er einen Wärmeaustauschkoeffizienten aufweist, der geringer als der Wärmeaustauschkoeffizient des ersten Abschnitts (100) ist.

2. Austauscher nach Anspruch 1, **dadurch gekennzeichnet, dass** der erste und zweite Abschnitt (100, 200) jeweils mindestens eine erste Welle und mindestens eine zweite Welle umfassen, wobei die erste und zweite Welle entlang der Längsachse (z) nebeneinandergestellt sind und jeweils mehrere Wellenschenkel (123, 223) aufweisen, die abwechselnd durch Wellenberge (121, 221) und Wellentäler (122, 222) verbunden sind, die entlang einer Welligkeitsrichtung (D1, D2) aufeinanderfolgen, wobei sich die zweite Welle gegenüber mindestens einem Teil der Mischvorrichtung (3) erstreckt und derart konfiguriert ist, dass sie einen Wärmeaustauschkoeffizienten aufweist, der geringer als der Wärmeaustauschkoeffizient der ersten Welle ist.

3. Austauscher nach Anspruch 2, **dadurch gekennzeichnet, dass** die zweite Welle eine Dichte aufweist, die geringer als die Dichte der ersten Welle ist, wobei die Dichte jeder Welle als die Anzahl der Wellenschenkel je gemessener Längeneinheit entlang der Welligkeitsrichtung (D1, D2) definiert ist.

4. Austauscher nach Anspruch 3, **dadurch gekennzeichnet, dass** die Dichte der zweiten Welle zwischen 20 % und 80 %, vorzugsweise zwischen 25 % und 50 % der Dichte der ersten Welle darstellt.

5. Austauscher nach einem der Ansprüche 3 oder 4, **dadurch gekennzeichnet, dass** die Dichte der zweiten Welle zwischen 6 und 15 Schenkeln je Zoll (1 Zoll = 2,54 Zentimeter) enthalten ist.

6. Austauscher nach einem der vorstehenden Ansprüche, **dadurch gekennzeichnet, dass** der erste und zweite Abschnitt (100, 200) aus Bändern gebildet sind, wobei der zweite Abschnitt (200) aus mindestens einem Band gebildet ist, das eine Dicke aufweist, die größer als die Dicke mindestens eines Bandes ist, das die erste Welle des Abschnitts (100) bildet.

7. Austauscher nach einem der Ansprüche 2 bis 6, **dadurch gekennzeichnet, dass** die zweite Welle eine gerade Welle ist, wobei die erste Welle aus einer geraden Welle, einer perforierten geraden Welle, einer Welle mit Teilversatz, einer Schwallwelle oder einer Welle vom Typ "mit Fischgräten" ausgewählt ist.

8. Austauscher nach einem der vorstehenden Ansprüche, **dadurch gekennzeichnet, dass** sich der zweite Abschnitt (200) mindestens gegenüber der Gesamtheit der Mischvorrichtung (3) erstreckt.

9. Austauscher nach einem der vorstehenden Ansprüche, **dadurch gekennzeichnet, dass** der zweite Abschnitt (200) parallel zur Längsachse (z) eine Länge aufweist, die zwischen 30 und 500 mm enthalten ist.

10. Austauscher nach einem der vorstehenden Ansprüche, **dadurch gekennzeichnet, dass** die Wärmetauschstruktur weiter entlang der Längsachse (z) in einen dritten Abschnitt (300) geteilt ist, wobei der zweite Abschnitt (200) derart konfiguriert ist, dass er einen Wärmetauschkoeffizienten aufweist, der kleiner oder gleich dem Wärmetauschkoeffizienten des dritten Abschnitts (300) ist, wobei der zweite Abschnitt (200) zwischen dem ersten Abschnitt (100) und dem dritten Abschnitt (300) angeordnet ist.

11. Austauscher nach einem der vorstehenden Ansprüche, **dadurch gekennzeichnet, dass** er an einem selben Ende des Austauschers erste Mittel (42) zum Verteilen oder Auslassen des wärmeerzeugenden Fluids (F2) in den oder aus dem mindestens einen Durchlass der zweiten Reihe von Durchlässen (20) und zweite Mittel (52) zum Verteilen einer flüssigen oder gasförmigen Phase des Kältemittelfluids (F1) in den mindestens einen Durchlass (10) der ersten Reihe zu der Mischvorrichtung (3) umfasst.

12. Austauscher nach einem der vorstehenden Ansprüche, **dadurch gekennzeichnet, dass** die Mischvorrichtung (3) unterschiedliche Einlässe (31, 32) für eine flüssige Phase (61) und eine gasförmige Phase (62) des Kältemittelfluids (F1) umfasst, wobei die unterschiedlichen Einlässe (31, 32) fluidisch über ein gemeinsames Mischervolumen mit mindestens einem Ausgang (33) für ein Gemisch der flüssigen und gasförmigen Phase (61, 62) verbunden sind.

13. Austauscher nach Anspruch 12, **dadurch gekennzeichnet, dass** sich der Ausgang (33) für ein Gemisch mit zwei Phasen flüssig/gasförmig der Mischvorrichtung (3) entlang der Längsachse (z) in einer ersten Position (z1) in dem mindestens einen Durchlass (10) der ersten Reihe befindet, wobei sich der zweite Abschnitt (200) mindestens aus der ersten Position (z1) bis zu einer zweiten Position (z2) erstreckt, die sich zwischen der ersten Position (z1) und den ersten Mitteln (42) zum Verteilen oder Auslassen des wärmeerzeugenden Fluids (F2) befindet.

14. Austauscher nach einem der Ansprüche 11 bis 13, **dadurch gekennzeichnet, dass** sich der zweite Abschnitt (200) bis zu den ersten Mitteln (42) zum Verteilen oder Auslassen des wärmeerzeugenden Fluids (F2) erstreckt.

15. Austauscher nach einem der Ansprüche 11 bis 14, **dadurch gekennzeichnet, dass** der zweite Abschnitt (200) aus den gesamten ersten Mitteln (42) zum Verteilen oder Auslassen des wärmeerzeugenden Fluids (F2) oder einem Teil davon gebildet ist.

**Claims**

1. Heat exchanger (1) comprising:

   - several plates (2) arranged parallel to one another so as to define a first series of passages (10) to channel at least one refrigerant fluid (F1) and a second series of passages (20) to channel at least one calorigenic fluid (F2) to put through heat exchange with at least said refrigerant fluid (F1), each passage (10, 20) being defined between two successive plates (2) and extending parallel to a longitudinal axis (z), and
   - at least one mixing device (3) arranged in at least one passage (10) of the first series, said mixing device (3) being configured to receive a liquid phase (61) and a gaseous phase (62) of the refrigerant fluid (F1) and distribute a mixture of said phases (61, 62) in said at least one passage (10),
   - at least one passage (20) of the second series adjacent to said at least one passage (10) of the first series comprising a divided heat exchange structure, along the longitudinal axis (z), in at least one first portion (100) and a second portion (200) juxtaposed along the longitudinal axis (z), the second portion (200) extending facing at least one portion of the mixing device (3),

   **characterised in that** the second portion (200) is configured so as to have a heat exchange coefficient less than the heat exchange coefficient of the first portion (100).

2. Exchanger according to claim 1, **characterised in that** said first and second portions (100, 200) comprise respectively at least one first wave and at least one second wave, said first and second waves being juxtaposed along the longitudinal axis (z) and each having several wave struts (123, 223) connected alternatively by wave crests (121, 221) and by wave bases (122, 222) succeeding one another along a wave direction (D1, D2), the second wave extending facing at least one portion of the mixing device (3) and being configured to as to have a heat exchange coefficient less than the heat exchange coefficient of the first wave.

3. Exchanger according to claim 2, **characterised in that** the second wave has a density less than the density of the first wave, the density of each wave being defined as the number of wave struts per unit of length measured along the wave direction (D1, D2).

4. Exchanger according to claim 3, **characterised in that** the density of the second wave represents between 20% and 80%, preferably between 25% and 50%, of the density of the first wave.

5. Exchanger according to one of claims 3 or 4, **characterised in that** the density of the second wave is between 6 and 15 struts per inch (1 inch = 2.54 centimetres).

6. Exchanger according to one of the preceding claims, **characterised in that** said first and second portions (100, 200) are formed from strips, the second portion (200) being formed from at least one strip having a thickness greater than the thickness of at least one strip forming said first wave of the portion (100).

7. Exchanger according to one of claims 2 to 6, **characterised in that** the second wave is a straight wave, the first wave being selected from among a straight wave, a perforated straight wave, a partially offset wave, a ripple wave or a wave of the "herringbone" type.

8. Exchanger according to one of the preceding claims, **characterised in that** the second portion (200) extends at least facing all of the mixing device (3).

9. Exchanger according to one of the preceding claims, **characterised in that** the second portion (200) has, parallel to the longitudinal axis (z), a length between 30 and 500mm.

10. Exchanger according to one of the preceding claims, **characterised in that** the heat exchange structure is furthermore divided, along the longitudinal axis (z), in a third portion (300), the second portion (200) being configured so as to have a heat exchange coefficient less than or equal to the heat exchange coefficient of the third portion (300), the second portion (200) being arranged between the first portion (100) and the third portion (300).

11. Exchanger according to one of the preceding claims, **characterised in that** it comprises, at one same end of the exchanger, first means (42) for distributing or evacuating the calorigenic fluid (F2) in said or of said at least one passage of the second series of passages (20) and second means (52) for distributing a liquid or gaseous phase of the refrigerant fluid (F1) in said at least one passage (10) from the first series to the mixing device (3).

12. Exchanger according to one of the preceding claims, **characterised in that** the mixing device (3) comprises separate inlets (31, 32) for a liquid phase (61) and a gaseous phase (62) of the refrigerant fluid (F1), said separate inlets (31, 32) being fluidically connected, via a common mixing volume, to at least one outlet (33) for a mixture of said liquid and gaseous phases (61, 62).

13. Exchanger according to claim 12, **characterised in that** the outlet (33) for a liquid/gas two-phase mixture of the mixing device (3) is situated, along the longitudinal axis (z), at a first position (z1) in said at least one passage (10) of the first series, the second portion (200) extending at least from the first position (z1) to a second position (z2) situated between the first position (z1) and the first means (42) for distributing or evacuating the calorigenic fluid (F2).

14. Exchanger according to one of claims 11 to 13, **characterised in that** the second portion (200) extends to the first means (42) for distributing or evacuating the calorigenic fluid (F2).

15. Exchanger according to one of claims 11 to 14, **characterised in that** the second portion (200) is formed by all or some of said first means (42) for distributing or evacuating the calorigenic fluid (F2).

Figure 1

31    3

34    32

Z

61

Figure 2A

34

31    33

62    F1

Z

35    36

32

Figure 2B

Figure 3

Figure 4

Figure 5A

Figure 5B

**RÉFÉRENCES CITÉES DANS LA DESCRIPTION**

**Documents brevets cités dans la description**

- FR 2563620 A